(19) Europäisches Patentamt
European Patent Office
Office européen des brevets

(11) **EP 2 604 327 A1**

(12) **EUROPEAN PATENT APPLICATION**
published in accordance with Art. 153(4) EPC

(43) Date of publication:
**19.06.2013 Bulletin 2013/25**

(51) Int Cl.:
**B01D 53/38** (2006.01)    **A61L 9/01** (2006.01)
**A61L 9/16** (2006.01)    **B01D 53/81** (2006.01)
**B01J 20/20** (2006.01)

(21) Application number: **11816487.0**

(22) Date of filing: **11.08.2011**

(86) International application number:
**PCT/JP2011/068356**

(87) International publication number:
**WO 2012/020824 (16.02.2012 Gazette 2012/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.02.2011   JP 2011042056**
**28.02.2011   JP 2011042058**
**11.08.2010   JP 2010180093**

(71) Applicant: **Toyobo Co., Ltd.**
**Osaka-shi**
**Osaka 530-8230 (JP)**

(72) Inventor: **IWASAKI,Keiko**
**Ohtsu-shi**
**Shiga 520-0292 (JP)**

(74) Representative: **Müller-Boré & Partner**
**Patentanwälte**
**Grafinger Straße 2**
**81671 München (DE)**

(54)    **FLAME-RETARDANT DEODORIZING FILTER**

(57)    [problem] It is provided that an activated carbon sheet to be excellent in removability of hazardous gas component and flame retardancy.

[solution] A flame-retardant deodorizing filter comprising an activated carbon layer and a fabric formed on both sides thereof, wherein the fabric has a fiber ratio of one or more kinds selected from cellulose fibers, polyvinyl alcohol fibers, polyacrylonitrile fibers, and phenol fibers in 30% or more; a phosphorus-based flame retardant is contained in an amount of 10 to 70% by weight relative to the fabric weight; an adhesive layer is present between the activated carbon layer and the fabric; the activated carbon particles are firmly adhered by thermoplastic resin binder particles; and the weight per unit area of activated carbon is 50 to 400 g/m$^2$.

FIG.1

EP 2 604 327 A1

Description

TECHNICAL FIELD

The present invention relates to a flame-retardant deodorizing filter for removing a hazardous gas component while being installed in an electronic appliance such as a copy machine, a printer, a multi-functional OA appliance, a computer, a projector, or a POD printer. Herein, the hazardous gas component may include not only a hazardous gas component affecting on a human body or an environment but also a gas and the like which causes a problem in the inside of an electronic appliance.

BACKGROUND ART

In recent years, integration and miniaturization of an electronic appliance such as a copy machine, a printer, a multi-functional OA appliance, a computer, a projector, or a POD printer has been advanced and in order to avoid heat accumulation in the inside of the electronic appliance, heat dissipation by a fan or the like has become indispensable. Consequently, various kinds of components, e.g., components such as ink and toner to be used at the time of printing; plastics constituting main bodies of electronic appliances; rubber used for various joining parts; and the like are gasified and discharged as hazardous gas components together with heat to be dissipated to the inside of a room. Moreover, since a copy machine, a laser printer, or the like uses a high voltage, besides the above-mentioned gas component, another hazardous gas component such as ozone is also discharged. Due to an increase in consciousness on environmental issues in recent years, emission of a hazardous gas component has been regulated. For example, in Germany, an environmental label called as "BAM (Blue Angel Mark)" is enacted and an environmental functional standard to be satisfied is determined for every electronic appliance.
For the purpose of lowering a hazardous gas component discharged from an electronic appliance, a deodorizing filter is installed in the inside of the electronic appliance. Since being installed in the inside of the electronic appliance, the above-mentioned deodorizing filter is indispensable not only to be excellent in removal of the hazardous gas component but also to gain fire retardant standard: UL (Underwriters Laboratories Inc.) (see Non-Patent Document 1).
A flame-retardant ozone/VOC removal filter for removing ozone and VOC while being installed in electronic appliances including a laser printer, various air conditioners, or the like has been known well. For example, Patent Document 1 describes a layered-type deodorizing filter medium obtained by depositing two or more different kinds of deodorization particulates on a cover material made of a fabric. However, regarding the filter medium in Patent Document 1, fiber webs exist only on one side thereof and the hot melt resin bonding the fibers is melted and the deodorization particulates drip as a flammable substance at the time of combustion, and thus there is a problem that the flame retardancy is insufficient.
Adsorption sheets obtained by sandwiching an adsorbent and a thermoplastic resin between substrate layers have also been disclosed (see, for example, Patent Documents 2 and 3). These adsorption sheets have substrate layers on the top and bottom of the adsorption layer; however, since these adsorption sheets contain no flame retardant in the substrate material, the substrate material is melted at the time of combustion and further, the thermoplastic resin mixed with the adsorbent is melted and thus the adsorbent drips as a flammable substance and there is a problem that the flame retardancy is insufficient.
Patent Document 4 describes that potassium carbonate is impregnated with activated carbon in order to improve the ozone removal capability of the activated carbon; however, an alkali metal compound or an alkaline earth metal impregnated for assisting the ozonolysis function promote combustion of the activated carbon at the time of combustion, and since a cover material is melted and an adsorbent drips as a flammable substance and thus there is a problem that the flame retardancy is insufficient.
Patent Document 5 describes that activated carbon and activated carbon or activated carbon and a cover material are bonded or firmly adhered by a hot melt resin; however, in the case of sheet formation using a hot melt resin or a nonwoven fabric, since adhesion of activated carbon and activated carbon is insufficient, there are problems that the activated carbon drops in a large amount at the time of normal use and that the activated carbon drips as a flammable substance at the time of combustion, resulting in failure of obtaining sufficient flame retardancy. Herein, "at the time of normal use" refers to use at a temperature condition of -20 to 80°C.
As described above, with respect to a deodorizing filter for removing a hazardous gas component while being installed in an electronic appliance such as a copy machine, a printer, a multi-functional OA appliance, a computer, a projector, or a POD printer, a material including activated carbon with a large specific surface area is used for the purpose of adsorbing VOC as an adsorbent, and for improvement of chemical ozone treatment performance, a technique of impregnating various kinds of chemical substances is employed. Although it is expected to improve flame retardancy by adding a flame retardant to a filter medium, the amount of a chemical substance impregnated is limited and satisfactory ozone treatment performance cannot be obtained.

If an activated carbon layer does not contain thermoplastic resin binder particles, not only activated carbon particles drop at the time of normal use but also the activated carbon particles drip as a flammable substance in a combustion, and thus there is a problem that sufficient flame retardancy cannot be obtained.

PRIOR ART DOCUMENTS

PATENT DOCUMENTS

Patent Document 1: JP-A-Hei-11-57467
Patent Document 2: JP-A-08-206550
Patent Document 3: JP-A-07-301434
Patent Document 4: JP-A-08-114109
Patent Document 5: JP-A-Hei-11-57467

NON-PATENT DOCUMENT

Non-Patent Document 1: UL 94:Standard for Safety Tests for Flammability of Plastic Materials for Parts in Devices and Appliances.

SUMMARY OF THE INVENTION

PROBLEMS TO BE SOLVED BY THE INVENTION

The present invention has been made in view of the state of the above conventional art, and it is an object of the present invention to provide a deodorizing filter which can remove a hazardous gas component while being installed in an electronic appliance such as a copy machine, a printer, a multi-functional OA appliance, a computer, a projector, or a POD printer and which has sufficient flame retardancy at the time of combustion.

SOLUTIONS TO THE PROBLEMS

The inventor of the present invention has made various investigations for accomplishing the above-mentioned object, and consequently found that a cover material is not melted at the time of combustion and activated carbon particles are suppressed in dropping off from an activated carbon layer by using a fabric containing fibers which are carbonized at the time of combustion as a cover material and making the cover material flame retardant, and the finding has led to completion of the prevent invention.
In other words, the present invention is as follows.

1. A flame-retardant deodorizing filter comprising an activated carbon layer and a fabric formed on both sides thereof, wherein the fabric has a fiber ratio of one or more kinds selected from cellulose fibers, polyvinyl alcohol fibers, polyacrylonitrile fibers, and phenol fibers in 30% or more; a phosphorus-based flame retardant is contained in an amount of 10 to 70% by weight relative to the fabric weight; an adhesive layer is present between the activated carbon layer and the fabric; the activated carbon particles are firmly adhered by thermoplastic resin binder particles; and the weight per unit area of activated carbon is 50 to 400 $g/m^2$.
2. The flame-retardant deodorizing filter according to above 1, wherein the activated carbon layer contains the thermoplastic resin binder particles in an amount of 1 to 10% by weight relative to the activated carbon weight mixed with the activated carbon.
3. A flame-retardant deodorizing filter comprising an activated carbon layer and a fabric formed on both sides thereof, wherein the fabric has a fiber ratio of one or more kinds selected from cellulose fibers, polyvinyl alcohol fibers, polyacrylonitrile fibers, and phenol fibers in 30% or more; a phosphorus-based flame retardant is contained in an amount of 10 to 80% by weight relative to the fabric weight; the activated carbon particles are firmly adhered by polyester resin or polyamide resin binder particles; and the weight per unit area of activated carbon is 50 to 400 $g/m^2$.
4. The flame-retardant deodorizing filter according to above 3, wherein the activated carbon layer contains the polyester resin or polyamide resin binder particles in an amount of 1 to 15% by weight relative to the activated carbon weight mixed with the activated carbon.

EFFECT OF THE INVENTION

The flame-retardant deodorizing filter of the present invention can be a filter that has no dripping of activated carbon particles as a flammable substance at the time of combustion and that is provided with sufficient flame retardancy owing to a cover material which is not melted at the time of combustion; that has sufficient adhesiveness owing to thermoplastic resin binder particles; and that satisfies both of flame retardancy and VOC removal/ozonolysis performance.

**BRIEF DESCRIPTION OF THE DRAWINGS**

[Fig.1] Fig. 1 is schematic cross-sectional diagram of the flame-retardant deodorizing filter of Embodiment 1 of the present invention.
[Fig.2] Fig. 2 is schematic cross-sectional diagram of the flame-retardant deodorizing filter of Embodiment 2 of the present invention.
[Fig.3] Fig. 3 is schematic cross-sectional diagram of the flame-retardant deodorizing filter of Embodiment 3 of the present invention.

MODE FOR CARRYING OUT THE INVENTION

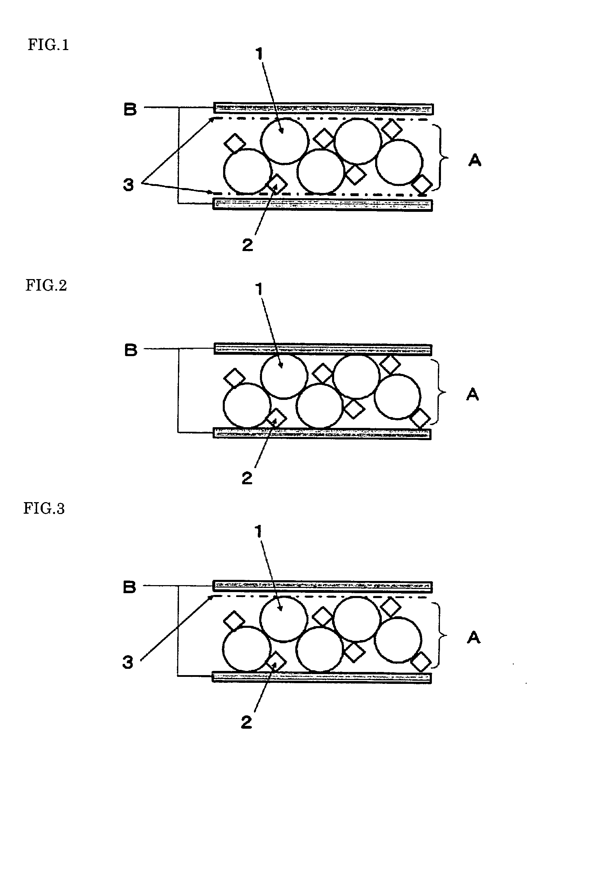

Hereinafter, the flame-retardant deodorizing filter of the present invention will be described in detail.
As shown in Fig. 1, the flame-retardant deodorizing filter of Embodiment 1 of the present invention includes an activated carbon layer A and a cover material B formed on both surfaces of the activated carbon layer A, and the cover material B contains fibers which are carbonized at the time of combustion and is made to be flame-retardant. In the activated carbon layer A, the activated carbon particles are firmly adhered by thermoplastic resin binder particles and further firmly adhered to the cover material B by an adhesive layer.
As shown in Fig. 2, the flame-retardant deodorizing filter of Embodiment 2 of the present invention includes an activated carbon layer A and a cover material B formed on both surfaces of the activated carbon layer A, and the cover material B contains fibers which are carbonized at the time of combustion and is made to be flame-retardant. In the activated carbon layer A, the activated carbon particles are firmly adhered by thermoplastic polyester resin or polyamide resin binder particles.
As shown in Fig. 3, the flame-retardant deodorizing filter of Embodiment 3 of the present invention includes an activated carbon layer A and a cover material B formed on both surfaces of the activated carbon layer A, and the cover material B contains fibers which are carbonized at the time of combustion and is made to be flame-retardant. In the activated carbon layer A, activated carbon particles are firmly adhered by thermoplastic polyester resin or polyamide resin binder particles and further firmly adhered to at least one side of the cover material B by an adhesive layer.
The activated carbon particles to be used for the activated carbon layer of the filter of the present invention are not particularly limited if the filter can remove a hazardous gas component, and for example, coal-based activated carbon, coconut shell-based activated carbon, wood-based activated carbon, and the like can be used. The specific surface area of the activated carbon particle to be used is preferably 1000 $m^2$/g or wider, and more preferably 1400 to 2000 $m^2$/g. If the specific surface area is narrower than 1000 $m^2$/g, toluene removal performance may possibly be lowered.
The average particle diameter of the activated carbon particles in the present invention is not particularly limited, but is preferably 50 to 850 $\mu$m. If the average particle diameter is smaller than 50 $\mu$m, powder dust and the like may be generated and the handling property may be worsened, and if it exceeds 850 $\mu$m, formation of the activated carbon layer may possibly become difficult.
Impregnated activated carbon particles impregnated with an alkali metal compound and an alkaline earth metal compound for assisting the ozonolysis function may be used as the activated carbon particles of the present invention.
Specific examples of the alkali metal compound and the alkaline earth metal compound include, as the alkali metal compound, salts in aqueous solutions such as hydroxides, oxides, carbonates, hydrogencarbonates, acetates, oxalates, phosphates, sulfates, succinates, phthalates, and hydrogenphthalates and halides of alkali metals such as sodium, potassium, and lithium; and, as the alkaline earth metal compound, salts in aqueous solutions such as hydroxides, oxides, carbonates, hydrogencarbonates, acetates, oxalates, and phosphates and halides of alkaline earth metals such as calcium, magnesium, and barium.
The activated carbon layer of the prevent invention contains thermoplastic resin binder particles for adhering the respective components of the activated carbon layer and improving the handling property at the time of forming the activated carbon layer. Examples of the thermoplastic resin binder particles to be used in Embodiment 1 may include publicly known thermoplastic resins such as polyolefin resins (polyethylene resin, polypropylene resin, etc.), polyester resins, polystyrene resins, polyvinyl acetate, urea-based resins, acrylic resins, and polyamide resins. In terms of economy and availability, polyolefin resins and polyester resins are preferable among these resins.
Examples of the thermoplastic resin binder particles to be used in Embodiments 2 and 3 may include thermoplastic resins such as polyester resins, urea-based resins, polyamide resins, and polyimide resins with a high limiting oxygen index (LOI value), and in terms of economy and availability, polyester resins and polyamide resins are used.

The average particle diameter of the thermoplastic resin binder particles in the activated carbon layer is not particularly limited, and in Embodiment 1, it is preferably 1.0 to 100 $\mu$m, and more preferably 5.0 to 50 $\mu$m. If the average particle diameter is smaller than 1.0 $\mu$m, powder dust and the like may be generated and the handling property may be worsened, and if it exceeds 100 $\mu$m, the thermoplastic resin binder particles may not be mixed evenly with activated carbon.

In Embodiment 2 and 3, the average particle diameter of the thermoplastic resin binder particles in the activated carbon layer is preferably 1.0 to 150 $\mu$m, and more preferably 5.0 to 80 $\mu$m. If the average particle diameter is smaller than 1.0 $\mu$m, powder dust and the like may be generated and the handling property may be worsened, and if it exceeds 150 $\mu$m, the thermoplastic resin binder particles may not be mixed evenly with activated carbon.

The malting point of the thermoplastic resin binder particles in the activated carbon layer is preferably 80°C to 150°C in consideration of the ambient temperature of business machines or the like. It is more preferably 100°C to 130°C.

The fluidity of the thermoplastic resin binder particles in the activated carbon layer at the time of melting is preferably 1 to 80 g/10 minutes as an MI value described in JIS K 7210. It is more preferably 3 to 30 g/10 minutes. If it is within the range, the thermoplastic resin binder particles can firmly attach activated carbon and activated carbon while preventing coverage of the surface of activated carbon.

The content of the activated carbon particles in the activated carbon layer is 50 to 400 g/m$^2$, and preferably 100 to 300 g/m$^2$ in Embodiment 1. If the content of the activated carbon particles is lower than 50 g/m$^2$, the removal performance of a hazardous gas such as toluene is lowered, and if it exceeds 400 g/m$^2$, the action of activated carbon as a combustible substance is reinforced and therefore, the UL Standard cannot be satisfied.

The content of the activated carbon particles in the activated carbon layer is 50 to 400 g/m$^2$, and preferably 100 to 350 g/m$^2$, and more preferably 200 to 300 g/m$^2$ in Embodiments 2 and 3. If the content of the activated carbon particles is lower than 50 g/m$^2$, the removal performance of a hazardous gas such as toluene is lowered, and if it exceeds 400 g/m$^2$, the action of activated carbon as a combustible substance is reinforced and, therefore, the UL Standard cannot be satisfied.

The content of the thermoplastic resin binder particles in the activated carbon layer is preferably 1 to 10 parts by weight, and more preferably 3 to 7 parts by weight relative to 100 parts by weight of the activated carbon particles in Embodiment 1. If the content of the thermoplastic resin binder particles is lower than 1 part by weight, the firm adhering in the activated carbon layer may become weak and the handling property may be worsened, and if it exceeds 10 parts by weight, it may not become possible to satisfy both of the flame retardancy and the VOC removal/ozonolysis performance.

The content of the thermoplastic resin binder particles in the activated carbon layer is preferably 1 to 15 parts by weight, and more preferably 3 to 10 parts by weight relative to 100 parts by weight of the activated carbon particles in Embodiment 2 and 3. If the content of the thermoplastic resin binder particles is lower than 1 part by weight, the firm adhering in the activated carbon layer may become weak and the handling property may be worsened, and if it exceeds 15 parts by weight, it may not become possible to satisfy both of the flame retardancy and the VOC removal/ozonolysis performance.

The flame-retardant deodorizing filter of the present invention is required to have a fabric as a cover material on both surfaces of the activated carbon layer. Owing to such a layered structure, dropping off of an activated carbon in a combustion test can be suppressed. The fabric to be used is not particularly limited, and for example, a nonwoven fabric, a knitted material, and a woven fabric can be used, and a nonwoven fabric is preferable among them. In terms of prevention of dropping off of the activated carbon particles at the time of filter formation, those with meshes smaller than the particle diameter of the activated carbon particles are preferable.

The nonwoven fabric may be made from fibers containing one or more kinds of fibers selected from cellulose fibers, polyvinyl alcohol fibers, polyacrylonitrile fibers, and phenol fibers. In terms of the fiber shape retention at the time of combustion, the nonwoven fabric is one containing 30 to 100% of any of these fibers.

A method for producing the nonwoven fabric to be employed may be a thermal bonding method, a chemical bonding method, a needle punching method, a spunlace method (hydroentangling method), or the like. It is also preferable that the fabric is made from fibers which are kneaded with a flame retardant and spun.

The weight per unit area and thickness of the fabric are not particularly limited, and preferably 10 g/m$^2$ to 45 g/m$^2$ as the weight per unit area and 0.05 mm to 2 mm as the thickness. If the weight per unit area is lower than 10 g/m$^2$, the activated carbon particles may drop off from the fabric at the time of sheet formation process, and if it exceeds 45 g/m$^2$, the processibility at the time of activated carbon layer formation may possibly be worsened. If the thickness is thinner than 0.05 mm, the activated carbon particles may drop off from the fabric at the time of sheet formation process, and if it exceeds 2 mm, the handling property at the time of activated carbon layer formation may possibly be worsened. Herein, the thickness of the fabric refers to a thickness measured at a load of 7 gf/cm$^2$.

In the present invention, it is required that the fabric contains a flame retardant. The addition of a flame retardant to the fabric provides an effect that the fabric itself is made to be flame retardant and also an effect that the fiber shape of the fabric is kept at the time of combustion.

As the flame retardant to be contained in the fabric, a phosphorus-based flame retardant is preferable from the viewpoint of the flame-retardant effect, and a water-soluble flame retardant such as guanidine phosphate, ammonium phosphate, or ammonium polyphosphate is preferable in terms of application to the fabric and drying.

In Embodiment 1, the flame retardant is contained in the fabric in an amount of 10 to 80% by weight relative to the fabric weight. It is preferably 20 to 60% by weight. If the flame retardant is contained in an amount of less than 10% by weight, the flame retardancy of the entire of the filter may become insufficient, and if the flame retardant is contained exceeding 80% by weight, it may result in problems that the processibility for impregnating the fabric with the flame retardant is worsened and that the pressure loss is increased.

In Embodiments 2 and 3, the flame retardant is contained in the fabric in an amount of 10 to 80% by weight relative to the fabric weight. It is preferably 20 to 70% by weight. If the flame retardant is contained in an amount of less than 10% by weight, the flame retardancy of the entire of the filter may become insufficient, and if the flame retardant is contained exceeding 80% by weight, it may result in problems that the processibility for impregnating the fabric with the flame retardant is worsened and that the pressure loss is increased.

The adhesive layer may be a layer formed by carrying out dot-like printing on the fabric with a thermoplastic resin in a paste state, a layer formed by spraying a powder of a thermoplastic resin to the fabric, or a layer in a cobwebs-like hot melt nonwoven fabric obtained by melt-spinning of thermoplastic resin. The weight per unit area of the adhesive layer is preferably 5 to 50 $g/m^2$, and more preferably 10 to 30 $g/m^2$.

As the thermoplastic resin, thermoplastic polyamide-based resins, thermoplastic polyester resins, thermoplastic polyurethane resins, polyolefin resins, or modified polyolefin resins may be used solely or by mixing. Examples of the modified polyolefin resins mentioned herein include ethylene-vinyl acetate copolymers, saponified products of ethylene-vinyl acetate copolymers, ethylene-ethyl acrylate copolymers, ethylene-acrylic acid copolymers, ethylene-methacrylic acid copolymers, ethylene-maleic acid copolymers, ionomer resins (heat sensitive resins obtained by adding metals to ethylene-methacrylic acid copolymers), and the like. It is allowed that the thermoplastic resin contains a flame retardant; however, in order to obtain an excellent adhesiveness, it is preferable that no flame retardant is contained.

A method for layering the activated carbon layer and the fabric is not particularly limited, and examples thereof include a method using an adhesive, a method for layering and integrating the activated carbon layer and the fabric by using a hot melt nonwoven fabric or a hot melt resin such as thermoplastic resin binder particles, or the like. Examples of a method for layering and integrating the fabric and the activated carbon particles with an adhesive or a thermoplastic resin include a method for layering the fabric with a hot melt nonwoven fabric and activated carbon particles and integrating the layered sheet by heating and processing, or the like; a method for producing, in advance, a fabric to which a thermobonding resin is attached, and sandwiching the activated carbon particles with the fabric to obtain a sheet. If the latter method for producing, in advance, a fabric to which a thermobonding resin is attached is employed, the method is advantageous for lessening troubles in production process, and thus is preferable.

The weight per unit area and thickness of the above-mentioned hot melt nonwoven fabric are not particularly limited, and preferably 5 to 20 $g/m^2$ as the weight per unit area and 0.05 to 0.5 mm as the thickness. If the weight per unit area exceeds 20 $g/m^2$, the pressure loss of the filter may possibly be increased. If the thickness exceeds 0.5 mm, the handling property at the time of activated carbon layer formation may possibly be worsened. Further, if the weight per unit area is less than 5 $g/m^2$ and the thickness is thinner than 0.05 mm, the adhesiveness may be inferior and the interlayer peeling may possibly occur. Herein, the thickness of the fabric refers to a thickness measured at a load of 7 $gf/cm^2$.

The peel strength of the flame-retardant deodorizing filter of the present invention is preferably 5 to 100 gf/cm. It is more preferably 15 to 80 gf/cm. If the peel strength is lower than 5 gf/cm, the adhesive force between the cover material and the activated carbon layer may be weak and interlayer peeling may possibly occur at the time of sheet formation.

The deodorizing filter of the present invention configured as described above does not cause dropping off of the activated carbon particles in the activated carbon layer not only at the time of normal use but also at the time of combustion, so that the deodorizing filter not only can effectively remove a hazardous gas component but also is provided with high flame retardancy satisfying UL Standard 94 HF-1.

EXAMPLES

Hereinafter, the action effects of the deodorizing filter of the present invention will be specifically described by way of examples; however, the present invention should not be limited thereto. Methods for evaluating characteristic values measured in the examples will be described below.

(Flame retardancy)

Evaluation was carried out by the horizontal combustion test described in Non-Patent Document 1. In this horizontal combustion test, a supporting wire-netting on which a specimen can be placed at a prescribed height is used and absorbent cotton (reference cotton) is placed at a distance of 175 ± 25 mm under the wire-netting. A specimen which is cut in a strip shape with a length of 150 ± 1 mm and a width of 50 ± 1 mm and on which two reference lines in total are previously drawn at positions of 60 mm and 125 mm from one rim part in the longitudinal direction is set on the wire-netting. In the combustion test, while being placed horizontally on the wire-netting, the rim part of the specimen is exposed to flames for 60 ± 1 seconds from a lower part of the wire-netting, and thereafter, the flames are apart from the specimen. Time is measured from that moment and three kinds of duration:

[a] duration until flames (remaining flames) disappear;
[b] duration until flames and red heat (afterglow) disappear; and
[c] duration until flames or the front line of red heat reach to the reference line at 125 mm or until combustion of the specimen or red heat stops before the reference line at 125 mm: are recorded. The evaluation test in the above-mentioned manner is repeated 5 times and evaluation is conducted according to two classifications: "94 HF-1" and "94 HF-2" as shown in Table 1 below.

Table 1

| standard condition | 94HF-1 | 94HF-2 |
|---|---|---|
| duration until remaining flames disappear | 2 seconds or less in four times out of five times, and 10 seconds or less in once out of five times | 2 seconds or less in four times out of five times, and 10 seconds or less in once out of five times |
| duration until afterglow disappear | 30 or less seconds | 30 or less seconds |
| combustion of reference cotton by dripping of flammable substance | no combustion | combustion |
| broken length of respective specimen | less than 60mm | less than 60mm |

(Toluene removal efficiency)

Air containing 5 ppm of toluene was passed at a velocity of 10 cm/second through a filter specimen and the toluene concentrations (ppm) in the inlet side and outlet side of a column were measured. The measurement condition was set at a temperature of 25°C and a humidity of 50%. The following formula was substituted with these measurement values to calculate the toluene removal efficiency (%). In order to measure the initial efficiency, the toluene removal efficiency after 1 minute from the starting of the measurement was measured.

$$\text{Toluene removal efficiency (\%)} = \{1 - (\text{the toluene concentrations (ppm) in the outlet side/the toluene concentrations (ppm) in the inlet side})\} \times 100$$

(Ozone removal efficiency)

Air containing 4 ppm of ozone was passed at a velocity of 10 cm/second through a filter specimen and the ozone concentrations (ppm) in the inlet side and outlet side of a column were measured. The measurement condition was set at a temperature of 25°C and a humidity of 50%. The following formula was substituted with these measurement values to calculate the ozone removal efficiency. In order to measure the initial efficiency, the ozone removal efficiency after 1 minute from the starting of the measurement was measured.

$$\text{Ozone removal efficiency (\%)} = \{1 - (\text{the ozone concentrations (ppm) in the outlet side/the ozone concentrations (ppm) in the inlet side})\} \times 100$$

(Average particle diameter)

The respective particles were observed with a scanning electron microscope (SEM), and the diameter of each of 100 particles was measured, and the average diameter was calculated from the measurement results.

(Adjustment of flame retardant)

A flame-retardant solution containing 50 parts of a phosphorus-based flame-retardant solution (an aqueous solution obtained by dissolving a flame retardant in water: dispersion concentration 60%) and 50 parts of water was prepared.

(Adhesiveness of sheet)

An activated carbon sheet was cut into a size of 50 mm × 150 mm and peeled previously about 50 mm in the direction of the longer side. Thereafter, a specimen was set in a tensile testing apparatus while the intervals of gripping a specimen were adjusted to 5 cm, and the peel strength of 100 mm distance was measured at a tensile speed of 100 mm/minute. The peel strength was defined as the average value of peel strength of 6 in total; 3 with highest maximum values and 3 with lowest maximum values.
The peel strength could be shown by a numeral value for a sample which showed neat peeling; however, a sample in which the cover material was stretched or cut during the test was shown as material failure. In the case of material failure, it shows that the adhesiveness of the activated carbon layer is higher than the peel strength of the cover material and the activated carbon layer, and, therefore, it means that the sheet adhesiveness is good.

(Preparation of cover material 1-1)

After spunlace nonwoven fabrics (weight per unit area 24 $g/m^2$, thickness 0.16 mm) of a mixture of rayon fibers/polyethylene terephthalate (hereinafter, referred to as "PET") fibers (weight ratio 70 : 30) were impregnated with the above-mentioned aqueous flame-retardant solution, the nonwoven fabrics were dried to obtain cover materials $(A_01)$, $(A_02)$, $(A_03)$, and $(A_04)$ with a weight per unit area of 26 $g/m^2$, 29 $g/m^2$, 34 $g/m^2$, and 43 $g/m^2$, respectively. The respective cover materials were cover materials impregnated with a phosphorus-based flame retardant on the surface of the spunlace fibers as the fabric, and the cover materials $(A_01)$, $(A_02)$, $(A_03)$, and $(A_04)$ contained a phosphorus-based flame retardant in an amount of 2 $g/m^2$, 5 $g/m^2$, 10 $g/m^2$, and 19 $g/m^2$, respectively.

(Preparation of cover material 1-2)

After spunlace nonwoven fabrics (weight per unit area 35 $g/m^2$, thickness 0.41 mm) of a mixture of rayon fibers/ PET fibers (weight ratio 70 : 30) were impregnated with the above-mentioned aqueous flame-retardant solution, the nonwoven fabrics were dried to obtain cover materials $(A_05)$, and $(A_06)$ with a weight per unit area of 40 $g/m^2$ and 55 $g/m^2$, respectively. The cover materials were cover materials impregnated with a phosphorus-based flame retardant on the surface of the spunlace fibers as the fabric, and the cover materials $(A_05)$ and $(A_06)$ contained a phosphorus-based flame retardant in an amount of 5 $g/m^2$ and 20 $g/m^2$, respectively.

(Preparation of cover material 1-3)

After spunlace nonwoven fabrics (weight per unit area 24 $g/m^2$, thickness 0.16 mm) of a mixture of rayon fibers/ PET fibers (weight ratio 10 : 90), spunlace nonwoven fabrics (weight per unit area 24 $g/m^2$, thickness 0.16 mm) of a mixture of rayon fibers/ PET fibers (weight ratio 40 : 60), and spunlace nonwoven fabrics (weight per unit area 24 $g/m^2$, thickness 0.16 mm) of rayon fibers were impregnated with the above-mentioned aqueous flame-retardant solution, the nonwoven fabrics were dried to obtain cover materials $(A_07)$, $(A_08)$, and $(A_09)$ with a weight per unit area of 34 $g/m^2$ respectively. The respective cover materials were cover materials impregnated with a phosphorus-based flame retardant on the surface of the spunlace fibers as the fabric, and the cover materials $(A_07)$, $(A_08)$, and $(A_09)$ contained a phosphorus-based flame retardant in an amount of 10 $g/m^2$ respectively.

(Preparation of cover material 2)

After a cobwebs-like hot melt nonwoven fabric with a weight per unit area of 10 $g/m^2$ was formed by melt-spinning of a thermoplastic polyamide-based resin, the nonwoven fabric was layered on the cover materials $(A_01)$, $(A_02)$, $(A_03)$, $(A_04)$, $(A_05)$, $(A_06)$, $(A_07)$, $(A_08)$, and $(A_09)$. The hot melt nonwoven fabric was cooled and simultaneously attached to the respective cover materials to obtain cover materials (A1) 36 g/m$^2$, (A2) 39 g/m$^2$, (A3) 44 g/m$^2$, (A4) 53 g/m$^2$, (A5) 50 g/m$^2$, (A6) 65 g/m$^2$, and (A7) to (A9) 44 g/m$^2$ to which the hot melt nonwoven fabric was attached.

(Example 1-1)

After an activated carbon layer was formed by spraying a mixture obtained by mixing activated carbon particles (average particle diameter 500 $\mu$m) in an amount of 250 g/m$^2$ and thermoplastic resin binder particles (average particle diameter 20 $\mu$m, polyethylene resin) in an amount of 12.5 g/m$^2$ onto the cover material (A2) prepared in the preparation of cover material 2, and the cover material (A2) was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 340.5 g/m$^2$.

(Example 1-2)

After an activated carbon layer was formed by spraying a mixture obtained by mixing activated carbon particles (average particle diameter 500 $\mu$m) in an amount of 250 g/m$^2$ and thermoplastic resin binder particles (average particle diameter 20 $\mu$m, polyethylene resin) in an amount of 12.5 g/m$^2$ onto the cover material (A3) prepared in the preparation of cover material 2, and the cover material (A3) was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 350.5 g/m$^2$.

(Example 1-3)

After an activated carbon layer was formed by spraying a mixture obtained by mixing activated carbon particles (average particle diameter 500 $\mu$m) in an amount of 250 g/m$^2$ and thermoplastic resin binder particles (average particle diameter 20 $\mu$m, polyethylene resin) in an amount of 12.5 g/m$^2$ onto the cover material (A4) prepared in the preparation of cover material 2, and the cover material (A4) was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 368.5 g/m$^2$.

(Example 1-4)

After an activated carbon layer was formed by spraying a mixture obtained by mixing activated carbon particles (average particle diameter 500 $\mu$m) in an amount of 250 g/m$^2$ and thermoplastic resin binder particles (average particle diameter 20 $\mu$m, polyethylene resin) in an amount of 12.5 g/m$^2$ onto the cover material (A8) prepared in the preparation of cover material 2, and the cover material (A8) was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 350.5 g/m$^2$.

(Example 1-5)

After an activated carbon layer was formed by spraying a mixture obtained by mixing activated carbon particles (average particle diameter 500 $\mu$m) in an amount of 250 g/m$^2$ and thermoplastic resin binder particles (average particle diameter 20 $\mu$m, polyethylene resin) in an amount of 12.5 g/m$^2$ onto the cover material (A9) prepared in the preparation of cover material 2, and the cover material (A9) was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 350.5 g/m$^2$.

(Example 1-6)

After an activated carbon layer was formed by spraying a mixture obtained by mixing activated carbon particles (average particle diameter 500 $\mu$m) in an amount of 100 g/m$^2$ and thermoplastic resin binder particles (average particle diameter 20 $\mu$m, polyethylene resin) in an amount of 5 g/m$^2$ onto the cover material (A2) prepared in the preparation of cover material 2, and the cover material (A2) was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 193 g/m$^2$.

(Example 1-7)

After an activated carbon layer was formed by spraying a mixture obtained by mixing activated carbon particles (average particle diameter 500 $\mu$m) in an amount of 350 g/m$^2$ and thermoplastic resin binder particles (average particle diameter 20 $\mu$m, polyethylene resin) in an amount of 17.5 g/m$^2$ onto the cover material (A2) prepared in the preparation of cover material 2, and the cover material (A2) was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 455.5 g/m$^2$.

(Example 1-8)

After an activated carbon layer was formed by spraying a mixture obtained by mixing activated carbon particles (average particle diameter 500 $\mu$m) in an amount of 250 g/m$^2$ and thermoplastic resin binder particles (average particle diameter 20 $\mu$m, polyethylene resin) in an amount of 7.5 g/m$^2$ onto the cover material (A2) prepared in the preparation of cover material 2, and the cover material (A2) was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 345.5 g/m$^2$.

(Example 1-9)

After an activated carbon layer was formed by spraying a mixture obtained by mixing activated carbon particles (average particle diameter 500 $\mu$m) in an amount of 250 g/m$^2$ and thermoplastic resin binder particles (average particle diameter 20 $\mu$m, polyethylene resin) in an amount of 17.5 g/m$^2$ onto the cover material (A2) prepared in the preparation of cover material 2, and the cover material (A2) was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 355.5 g/m$^2$.

(Example 1-10)

After an activated carbon layer was formed by spraying a mixture obtained by mixing activated carbon particles (average particle diameter 500 $\mu$m) in an amount of 250 g/m$^2$ and thermoplastic resin binder particles (average particle diameter 20 $\mu$m, polyethylene resin) in an amount of 12.5 g/m$^2$ onto the cover material (A5) prepared in the preparation of cover material 2, and the cover material (A5) was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 362.5 g/m$^2$.

(Example 1-11)

After an activated carbon layer was formed by spraying a mixture obtained by mixing activated carbon particles (average particle diameter 500 $\mu$m) in an amount of 250 g/m$^2$ and thermoplastic resin binder particles (average particle diameter 20 $\mu$m, polyethylene resin) in an amount of 12.5 g/m$^2$ onto the cover material (A6) prepared in the preparation of cover material 2, and the cover material (A6) was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 392.5 g/m$^2$.

(Example 1-12)

After an activated carbon layer was formed by spraying a mixture obtained by mixing activated carbon particles (average particle diameter 500 $\mu$m) in an amount of 100 g/m$^2$ and thermoplastic resin binder particles (average particle diameter 20 $\mu$m, polyethylene resin) in an amount of 7 g/m$^2$ onto the cover material (A8) prepared in the preparation of cover material 2, and the cover material (A8) was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 195 g/m$^2$.

(Example 1-13)

After an activated carbon layer was formed by spraying a mixture obtained by mixing activated carbon particles (average particle diameter 500 $\mu$m) in an amount of 300 g/m$^2$ and thermoplastic resin binder particles (average particle diameter 20 $\mu$m, polyethylene resin) in an amount of 21 g/m$^2$ onto the cover material (A8) prepared in the preparation of cover material 2, and the cover material (A8) was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 409 g/m$^2$.

(Comparative Example 1-1)

After an activated carbon layer was formed by spraying a mixture obtained by mixing activated carbon particles (average particle diameter 500 $\mu$m) in an amount of 100 g/m$^2$ and thermoplastic resin binder particles (average particle diameter 20 $\mu$m, polyethylene resin) in an amount of 5 g/m$^2$ onto the cover material (A2) prepared in the preparation of cover material 2, and the obtained layered product was sandwiched between iron plates heated to 140°C and heat-pressed for 1 minute to produce a filter having the activated carbon layer firmly adhered by the thermoplastic resin binder particles. In this case, the weight per unit area of the activated carbon layer was 159 g/m$^2$.

(Comparative Example 1-2)

After a cobwebs-like hot melt nonwoven fabric with a weight per unit area of 10 g/m$^2$ was formed by melt-spinning of a thermoplastic polyamide-based resin, the nonwoven fabric was layered on a spunlace nonwoven fabric (weight per unit area 24 g/m$^2$, thickness 0.16 mm) of a mixture of rayon fibers/PET fibers (weight ratio 70 : 30). The hot melt nonwoven fabric was cooled and simultaneously attached to the spunlace nonwoven fabric to obtain a spunlace nonwoven fabric to which the hot melt nonwoven fabric was attached.
After an activated carbon layer as formed by spraying a mixture obtained by mixing activated carbon particles (average particle diameter 500 $\mu$m) in an amount of 250 g/m$^2$ and thermoplastic resin binder particles (average particle diameter 20 $\mu$m, polyethylene resin) in an amount of 5 g/m$^2$ onto the spunlace nonwoven fabric, and the spunlace nonwoven fabric was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 173 g/m$^2$.

(Comparative Example 1-3)

After an activated carbon layer was formed by spraying a mixture obtained by mixing activated carbon particles (average particle diameter 500 $\mu$m) in an amount of 250 g/m$^2$ and thermoplastic resin binder particles (average particle diameter 20 $\mu$m, polyethylene resin) in an amount of 12.5 g/m$^2$ onto the cover material (A1) prepared in the preparation of cover material 2, and the cover material (A1) was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 334.5 g/m$^2$.

(Comparative Example 1-4)

After an activated carbon layer was formed by spraying a mixture obtained by mixing activated carbon particles (average particle diameter 500 $\mu$m) in an amount of 250 g/m$^2$ and thermoplastic resin binder particles (average particle diameter 20 $\mu$m, polyethylene resin) in an amount of 12.5 g/m$^2$ onto the cover material (A7) prepared in the preparation of cover material 2, and the cover material (A7) was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 350.5 g/m$^2$.

(Comparative Example 1-5)

After an activated carbon layer was formed by spraying a mixture obtained by mixing activated carbon particles (average particle diameter 500 $\mu$m) in an amount of 30 g/m$^2$ and thermoplastic resin binder particles (average particle diameter 20 $\mu$m, polyethylene resin) in an amount of 1.5 g/m$^2$ onto the cover material (A2) prepared in the preparation of cover material 2, and the cover material (A2) was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 119.5 g/m$^2$.

(Comparative Example 1-6)

After an activated carbon layer was formed by spraying a mixture obtained by mixing activated carbon particles (average particle diameter 500 $\mu$m) in an amount of 500 g/m$^2$ and thermoplastic resin binder particles (average particle diameter 20 $\mu$m, polyethylene resin) in an amount of 25 g/m$^2$ onto the cover material (A2) prepared in the preparation of cover material 2, and the cover material (A2) was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 613 g/m$^2$.
Table 2 shows the details of configuration and evaluation results of flame retardancy of the filters obtained in Examples 1-1 to 1-13 and Comparative Examples 1-1 to 1-6.
Table 2

| | cover material | | | | | adhesive layer | activated carbon layer | | | filter | | | |
|---|---|---|---|---|---|---|---|---|---|---|---|---|---|
| | upper layer | | lower layer | | ratio of flame retardant (relative to fabric) | weight per unit area | weight per unit area of activated carbon | weight per unit area of binder powder | ratio of binder powder (relative to activated carbon) | weight per unit area | initial removal capability | | flame retardancy test UL94HF |
| | weight per unit area of fiber | weight per unit area of flame retardant | weight per unit area of fiber | weight per unit area of flame retardant | | | | | | | toluene | ozone | |
| | g/m$^2$ | g/m$^2$ | g/m$^2$ | g/m$^2$ | weight % | g/m$^2$ | g/m$^2$ | g/m$^2$ | part by weight | g/m$^2$ | % | % | - |
| example 1-1 | 24 | 5 | 24 | 5 | 21 | 10×2 | 250 | 12.5 | 5 | 340.5 | 100 | 99 | HF-1 |
| example 1-2 | 24 | 10 | 24 | 10 | 42 | 10×2 | 250 | 12.5 | 5 | 350.5 | 100 | 99 | HF-1 |
| example 1-3 | 24 | 19 | 24 | 19 | 79 | 10×2 | 250 | 12.5 | 5 | 368.5 | 100 | 99 | HF-1 |
| example 1-4 | 24 | 10 | 24 | 10 | 42 | 10×2 | 250 | 12.5 | 5 | 350.5 | 100 | 99 | HF-1 |
| example 1-5 | 24 | 10 | 24 | 10 | 42 | 10×2 | 250 | 12.5 | 5 | 350.5 | 100 | 99 | HF-1 |
| example 1-6 | 24 | 10 | 24 | 10 | 42 | 10×2 | 100 | 5 | 5 | 193 | 80 | 95 | HF-1 |
| example 1-7 | 24 | 10 | 24 | 10 | 42 | 10×2 | 350 | 17.5 | 5 | 455.5 | 100 | 100 | HF-1 |
| example 1-8 | 24 | 10 | 24 | 10 | 42 | 10×2 | 250 | 7.5 | 3 | 345.5 | 100 | 100 | HF-1 |
| example 1-9 | 24 | 10 | 24 | 10 | 42 | 10×2 | 250 | 17.5 | 7 | 355.5 | 99 | 99 | HF-1 |
| example 1-10 | 35 | 5 | 35 | 5 | 14 | 10×2 | 250 | 12.5 | 5 | 362.5 | 100 | 99 | HF-1 |
| example 1-11 | 35 | 20 | 35 | 20 | 57 | 10×2 | 250 | 12.5 | 5 | 392.5 | 100 | 99 | HF-1 |
| example 1-12 | 24 | 10 | 24 | 10 | 42 | 10×2 | 100 | 7 | 7 | 195 | 78 | 92 | HF-1 |
| example 1-13 | 24 | 10 | 24 | 10 | 42 | 10×2 | 300 | 21 | 7 | 409 | 100 | 99 | HF-1 |

(continued)

| | cover material | | | | | adhesive layer | activated carbon layer | | | filter | | | |
|---|---|---|---|---|---|---|---|---|---|---|---|---|---|
| | upper layer | | lower layer | | ratio of flame retardant (relative to fabric) | weight per unit area | weight per unit area of activated carbon | weight per unit area of binder powder | ratio of binder powder (relative to activated carbon) | weight per unit area | initial removal capability | | flame retardancy test UL94HF |
| | weight per unit area of fiber | weight per unit area of flame retardant | weight per unit area of fiber | weight per unit area of flame retardant | | | | | | | | toluene | ozone | |
| comparative example i-1 | 24 | 10 | - | - | 42 | 10×2 | 100 | 5 | 5 | 159 | 80 | 95 | HF-2 |
| comparative example i-2 | 24 | - | 24 | - | 0 | 10×2 | 100 | 5 | 5 | 173 | 80 | 95 | HF-2 |
| comparative example 1-3 | 24 | 2 | 24 | 2 | 8 | 10×2 | 250 | 12.5 | 5 | 334.5 | 100 | 99 | HF-2 |
| comparative example 1-4 | 24 | 10 | 24 | 10 | 42 | 10×2 | 250 | 12.5 | 5 | 350.5 | 100 | 99 | HF-2 |
| comparative example 1-5 | 24 | 10 | 24 | 10 | 42 | 10×2 | 30 | 1.5 | 5 | 119.5 | 50 | 30 | HF-2 |
| comparative example 1-6 | 24 | 10 | 24 | 10 | 42 | 10×2 | 500 | 25 | 5 | 613 | 100 | 100 | HF-2 |

As is apparent from Table 2, Examples 1-1 to 1-13 are evaluated according to UL 94 HF-1 in the flame retardancy test, whereas Comparative Example 1-1 shows dropping off of the activated carbon at the time of combustion since the cover material is only on one side of the activated carbon layer, Comparative Examples 1-2 and 1-3 are insufficient in a flame-retardant effect to the cover material, Comparative Example 1-4 is improper in the ratio of fibers constituting the cover material, and Comparative Examples 1-5 and 1-6 have the weight per unit area of activated carbon beyond the upper and lower limits, and thus, these comparative examples do not have flame retardancy satisfying UL Standard 94 HF-1.

(Example 2-1)

After an activated carbon layer was formed by spraying a mixture obtained by mixing activated carbon particles (average particle diameter 500 $\mu$m) in an amount of 250 g/m$^2$ and thermoplastic resin binder particles (average particle diameter 50 $\mu$m, PET resin) in an amount of 12.5 g/m$^2$ onto the cover material ($A_0$2) prepared in the preparation of cover material 1, and the cover material (A2) was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 320.5 g/m$^2$.

(Example 2-2)

After an activated carbon layer was formed by spraying a mixture obtained by mixing activated carbon particles (average particle diameter 500 $\mu$m) in an amount of 250 g/m$^2$ and thermoplastic resin binder particles (average particle diameter 50 $\mu$m, PET resin) in an amount of 12.5 g/m$^2$ onto the cover material ($A_0$2) prepared in the preparation of cover material 1, and the cover material (A2) prepared in the preparation of cover material 2 was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 330.5 g/m$^2$.

(Example 2-3)

After an activated carbon layer was formed by spraying a mixture obtained by mixing activated carbon particles (average particle diameter 500 $\mu$m) in an amount of 250 g/m$^2$ and thermoplastic resin binder particles (average particle diameter 50 $\mu$m, polyamide resin) in an amount of 12.5 g/m$^2$ onto the cover material ($A_0$2) prepared in the preparation of cover material 1, and the cover material (A2) prepared in the preparation of cover material 2 was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 330.5 g/m$^2$.

(Example 2-4)

After an activated carbon layer was formed by spraying a mixture obtained by mixing activated carbon particles (average particle diameter 500 $\mu$m) in an amount of 250 g/m$^2$ and thermoplastic resin binder particles (average particle diameter 50 $\mu$m, PET resin) in an amount of 12.5 g/m$^2$ onto the cover material ($A_0$3) prepared in the preparation of cover material 1, and the cover material (A3) prepared in the preparation of cover material 2 was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 340.5 g/m$^2$.

(Example 2-5)

After an activated carbon layer was formed by spraying a mixture obtained by mixing activated carbon particles (average particle diameter 500 $\mu$m) in an amount of 250 g/m$^2$ and thermoplastic resin binder particles (average particle diameter 50 $\mu$m, PET resin) in an amount of 12.5 g/m$^2$ onto the cover material ($A_0$4) prepared in the preparation of cover material 1, and the cover material (A4) prepared in the preparation of cover material 2 was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 358.5 g/m$^2$.

(Example 2-6)

After an activated carbon layer was formed by spraying a mixture obtained by mixing activated carbon particles (average particle diameter 500 $\mu$m) in an amount of 250 g/m$^2$ and thermoplastic resin binder particles (average particle diameter 50 $\mu$m, PET resin) in an amount of 12.5 g/m$^2$ onto the cover material (A$_0$8) prepared in the preparation of cover material 1, and the cover material (A8) prepared in the preparation of cover material 2 was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 340.5 g/m$^2$.

(Example 2-7)

After an activated carbon layer was formed by spraying a mixture obtained by mixing activated carbon particles (average particle diameter 500 $\mu$m) in an amount of 250 g/m$^2$ and thermoplastic resin binder particles (average particle diameter 50 $\mu$m, PET resin) in an amount of 12.5 g/m$^2$ onto the cover material (A$_0$9) prepared in the preparation of cover material 1, and the cover material (A9) prepared in the preparation of cover material 2 was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 340.5 g/m$^2$.

(Example 2-8)

After an activated carbon layer was formed by spraying a mixture obtained by mixing activated carbon particles (average particle diameter 500 $\mu$m) in an amount of 100 g/m$^2$ and thermoplastic resin binder particles (average particle diameter 50 $\mu$m, PET resin) in an amount of 5 g/m$^2$ onto the cover material (A$_0$3) prepared in the preparation of cover material 1, and the cover material (A3) prepared in the preparation of cover material 2 was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 183 g/m$^2$.

(Example 2-9)

After an activated carbon layer was formed by spraying a mixture obtained by mixing activated carbon particles (average particle diameter 500 $\mu$m) in an amount of 350 g/m$^2$ and thermoplastic resin binder particles (average particle diameter 50 $\mu$m, PET resin) in an amount of 17.5 g/m$^2$ onto the cover material (A$_0$3) prepared in the preparation of cover material 1, and the cover material (A3) prepared in the preparation of cover material 2 was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 445.5 g/m$^2$.

(Example 2-10)

After an activated carbon layer was formed by spraying a mixture obtained by mixing activated carbon particles (average particle diameter 500 $\mu$m) in an amount of 250 g/m$^2$ and thermoplastic resin binder particles (average particle diameter 50 $\mu$m, PET resin) in an amount of 7.5 g/m$^2$ onto the cover material (A$_0$3) prepared in the preparation of cover material 1, and the cover material (A3) prepared in the preparation of cover material 2 was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 335.5 g/m$^2$.

(Example 2-11)

After an activated carbon layer was formed by spraying a mixture obtained by mixing activated carbon particles (average particle diameter 500 $\mu$m) in an amount of 250 g/m$^2$ and thermoplastic resin binder particles (average particle diameter 50 $\mu$m, PET resin) in an amount of 25 g/m$^2$ onto the cover material (A$_0$3) prepared in the preparation of cover material 1, and the cover material (A3) prepared in the preparation of cover material 2 was laminated on the activated carbon layer, the obtained layered product was sandwiched between iron plates heated to 140°C and heat-pressed for 1 minute to produce a filter having the activated carbon layer firmly adhered by the thermoplastic resin binder particles.

In this case, the weight per unit area of the filter was 353 g/m$^2$.

(Example 2-12)

After an activated carbon layer was formed by spraying a mixture obtained by mixing activated carbon particles (average particle diameter 500 $\mu$m) in an amount of 250 g/m$^2$ and thermoplastic resin binder particles (average particle diameter 50 $\mu$m, PET resin) in an amount of 12.5 g/m$^2$ onto the cover material (A$_0$5) prepared in the preparation of cover material 1, and the cover material (A5) prepared in the preparation of cover material 2 was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 352.5 g/m$^2$.

(Example 2-13)

After an activated carbon layer was formed by spraying a mixture obtained by mixing activated carbon particles (average particle diameter 500 $\mu$m) in an amount of 250 g/m$^2$ and thermoplastic resin binder particles (average particle diameter 50 $\mu$m, PET resin) in an amount of 12.5 g/m$^2$ onto the cover material (A$_0$6) prepared in the preparation of cover material 1, and the cover material (A6) prepared in the preparation of cover material 2 was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 382.5 g/m$^2$.

(Example 2-14)

After an activated carbon layer was formed by spraying a mixture obtained by mixing activated carbon particles (average particle diameter 500 $\mu$m) in an amount of 100 g/m$^2$ and thermoplastic resin binder particles (average particle diameter 50 $\mu$m, PET resin) in an amount of 10 g/m$^2$ onto the cover material (A$_0$8) prepared in the preparation of cover material 1, and the cover material (A8) prepared in the preparation of cover material 2 was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 188 g/m$^2$.

(Example 15)

After an activated carbon layer was formed by spraying a mixture obtained by mixing activated carbon particles (average particle diameter 500 $\mu$m) in an amount of 300 g/m$^2$ and thermoplastic resin binder particles (average particle diameter 50 $\mu$m, PET resin) in an amount of 30 g/m$^2$ onto the cover material (A$_0$8) prepared in the preparation of cover material 1, and the cover material (A8) prepared in the preparation of cover material 2 was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 408 g/m$^2$.

(Comparative Example 2-1)

After an activated carbon layer was formed by spraying a mixture obtained by mixing activated carbon particles (average particle diameter 500 $\mu$m) in an amount of 250 g/m$^2$ and thermoplastic resin binder particles (average particle diameter 50 $\mu$m, polyethylene resin) in an amount of 12.5 g/m$^2$ onto the cover material (A$_0$2) prepared in the preparation of cover material 1, and the cover material (A2) was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 320.5 g/m$^2$.

(Comparative Example 2-2)

After an activated carbon layer was formed by spraying a mixture obtained by mixing activated carbon particles (average particle diameter 500 $\mu$m) in an amount of 100 g/m$^2$ and thermoplastic resin binder particles (average particle diameter 50 $\mu$m, PET resin) in an amount of 5 g/m$^2$ onto the cover material (A$_0$3) prepared in the preparation of cover material 1, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 149 g/m$^2$.

(Comparative Example 2-3)

After a cobwebs-like hot melt nonwoven fabric with a weight per unit area of 10 g/m$^2$ was formed by melt-spinning of a thermoplastic polyamide-based resin, the nonwoven fabric was layered on the spunlace nonwoven fabrics (weight per unit area 24 g/m$^2$, thickness 0.16 mm) of a mixture of rayon fibers/ PET fibers (weight ratio 70 : 30). The hot melt nonwoven fabric was cooled and simultaneously attached to the spunlace nonwoven fabrics to obtain spunlace nonwoven fabrics to which the hot melt nonwoven fabric was attached.
After an activated carbon layer was formed by spraying a mixture obtained by mixing activated carbon particles (average particle diameter 500 $\mu$m) in an amount of 100 g/m$^2$ and thermoplastic resin binder particles (average particle diameter 50 $\mu$m, polyethylene resin) in an amount of 5 g/m$^2$ spunlace nonwoven fabrics before layering hot melt nonwoven fabric, and the spunlace nonwoven fabrics was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 163 g/m$^2$.

(Comparative Example 2-4)

After an activated carbon layer was formed by spraying a mixture obtained by mixing activated carbon particles (average particle diameter 500 $\mu$m) in an amount of 250 g/m$^2$ and thermoplastic resin binder particles (average particle diameter 50 $\mu$m, PET resin) in an amount of 12.5 g/m$^2$ onto the cover material ($A_0$1) prepared in the preparation of cover material 1, and the cover material (A1) prepared in the preparation of cover material 2 was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 324.5 g/m$^2$.

(Comparative Example 2-5)

After an activated carbon layer was formed by spraying a mixture obtained by mixing activated carbon particles (average particle diameter 500 $\mu$m) in an amount of 250 g/m$^2$ and thermoplastic resin binder particles (average particle diameter 50 $\mu$m, PET resin) in an amount of 12.5 g/m$^2$ onto the cover material ($A_0$7) prepared in the preparation of cover material 1, and the cover material (A7) prepared in the preparation of cover material 2 was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 340.5 g/m$^2$.

(Comparative Example 2-6)

After an activated carbon layer was formed by spraying a mixture obtained by mixing activated carbon particles (average particle diameter 500 $\mu$m) in an amount of 500 g/m$^2$ and thermoplastic resin binder particles (average particle diameter 50 $\mu$m, PET resin) in an amount of 25 g/m$^2$ onto the cover material ($A_0$2) prepared in the preparation of cover material 1, and the cover material (A2) prepared in the preparation of cover material 2 was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 603 g/m$^2$.

(Comparative Example 2-7)

After an activated carbon layer was formed by spraying a mixture obtained by mixing activated carbon particles (average particle diameter 500 $\mu$m) in an amount of 250 g/m$^2$ and thermoplastic resin binder particles (average particle diameter 50 $\mu$m, PET resin) in an amount of 1.25 g/m$^2$ onto the cover material ($A_0$2) prepared in the preparation of cover material 1, and the cover material (A2) prepared in the preparation of cover material 2 was laminated on the activated carbon layer, the obtained layered product was sandwiched between iron plates heated to 140°C and heat-pressed for 1 minute to produce a filter having the activated carbon layer firmly adhered by the thermoplastic resin binder particles. In this case, the weight per unit area of the filter was 329.3 g/m$^2$.
Table 3 shows the details of configuration and evaluation results of flame retardancy of the filters obtained in Examples 2-1 to 2-15 and Comparative Examples 2-1 to 2-7.
Table 3

| | clover material | | | | | adhesive layer | activated carbon layer | | | | filter | | | | |
|---|---|---|---|---|---|---|---|---|---|---|---|---|---|---|---|
| | upper layer | | lower layer | | ratio of flame retardant (relative to fabric) | weight per unit area | weight per unit area of activated carbon | kind of binder | weight per unit area of binder powder | ratio of binder powder (relative to activated carbon) | weight per unit area | Initial removal capability | | peel strength (peel or no peel) | flame retardancy test UL94HF |
| | weight per unit erea of fiber | weight per unit area of flame retardant | weight per unit area of fiber | weight per unit area of flame retardant | | | | | | | | toluene | ozone | | |
| | g/m² | g/m² | g/m² | g/m² | weight % | g/m² | g/m² | - | g/m² | part by weight | g/m² | % | % | gf/cm | - |
| example 2-1 | 24 | 5 | 24 | 5 | 21 | - | 250 | PET | 12.5 | 5 | 320.5 | 100 | 99 | 25(no Peel) | HF-1 |
| example 2-2 | 24 | 5 | 24 | 5 | 21 | 10×1 | 250 | PET | 12.5 | 5 | 330.5 | 100 | 99 | 35(no Peel) | HF-1 |
| example 2-3 | 24 | 5 | 24 | 5 | 21 | 10×1 | 250 | port amide | 12.5 | 5 | 330.5 | 100 | 99 | 35(no peel) | HF-1 |
| example 2-4 | 24 | 10 | 24 | 10 | 42 | 10×1 | 250 | PET | 12.5 | 5 | 340.5 | 100 | 89 | 35(no peel) | HF-1 |
| example 2-5 | 24 | 19 | 24 | 19 | 79 | 10×1 | 250 | PET | 12.5 | 5 | 358.5 | 100 | 99 | 35(no peel) | HF-1 |
| example 2-6 | 24 | 10 | 24 | 10 | 42 | 10×1 | 250 | PET | 12.5 | 5 | 340.5 | 100 | 99 | 35(no Peel) | HF-1 |
| example 2-7 | 24 | 10 | 24 | 10 | 42 | 10×1 | 250 | PET | 12.5 | 5 | 340.5 | 100 | 99 | 35(not peel) | HF-1 |
| example 2-8 | 24 | 10 | 24 | 10 | 42 | 10×1 | 100 | PET | 5 | 5 | 183 | 80 | 95 | 50(no peel) | HF-1 |
| example 2-9 | 24 | 10 | 24 | 10 | 42 | 10×1 | 350 | PET | 17.5 | 5 | 445.5 | 100 | 100 | 25(no Peel) | HF-1 |

| | clover material | | | | | adhesive layer | activated carbon layer | | | | filter | | | | |
| | upper layer | | lower layer | | ratio of flame retardant (relative to fabric) | weight per unit area | weight per unit area of activated carbon | kind of binder | weight per unit area of binder powder | ratio of binder powder (relative to activated carbon) | weight per unit area | Initial removal capability | | peel strength (peel or no peel) | flame retardancy test UL94HF |
| | weight per unit erea of fiber | weight per unit area of flame retardant | weight per unit area of fiber | weight per unit area of flame retardant | | | | | | | | toluene | ozone | | |
| example 2-10 | 24 | 10 | 24 | 10 | 42 | 10×1 | 250 | PET | 7.5 | 3 | 335.5 | 100 | 100 | 20(no peel) | HF-1 |
| example 2-11 | 24 | 10 | 24 | 10 | 42 | 10×1 | 250 | PET | 25 | 10 | 353 | 99 | 99 | 50(no peel) | HF-1 |
| example 2-12 | 35 | 5 | 35 | 5 | 14 | 10×1 | 250 | PET | 12.5 | 5 | 352.5 | 100 | 99 | 30(no peel) | HF-1 |
| example 2-13 | 35 | 20 | 35 | 20 | 57 | 10×1 | 250 | PET | 12.5 | 5 | 382.5 | 100 | 99 | 30(no peel) | HF-1 |
| example 2-14 | 24 | 10 | 24 | 10 | 42 | 10×1 | 100 | PET | 10 | 10 | 188 | 78 | 92 | 60(no peel) | HF-1 |
| example 2-15 | 24 | 10 | 24 | 10 | 42 | 10×1 | 300 | PET | 30 | 10 | 408 | 100 | 99 | 25(no peel) | HF-2 |
| comparative example 2-1 | 24 | 5 | 24 | 5 | 21 | - | 250 | PE | 12.5 | 5 | 320.5 | 100 | 99 | 48(no peel) | HF-2 |
| comparative example 2-2 | 24 | 10 | - | - | 42 | 10×1 | 100 | PET | 5 | 5 | 149 | 80 | 95 | - | HF-2 |

EP 2 604 327 A1

(continued)

| | clover material | | | | ratio of flame retardant (relative to fabric) | adhesive layer | activated carbon layer | | | | filter | | | | |
|---|---|---|---|---|---|---|---|---|---|---|---|---|---|---|---|
| | upper layer | | lower layer | | | weight per unit area | | | | | | Initial removal capability | | peel strength (peel or no peel) | flame retardancy test UL94HF |
| | weight per unit erea of fiber | weight per unit area of flame retardant | weight per unit area of fiber | weight per unit area of flame retardant | | | weight per unit area of activated carbon | kind of binder | weight per unit area of binder powder | ratio of binder powder (relative to activated carbon) | weight per unit area | toluene | ozone | | |
| comparative exemple 2-3 | 24 | - | 24 | - | 0 | 10×1 | 100 | PET | 5 | 5 | 163 | 80 | 95 | 45(no peel) | HF-2 |
| comparative example 2-4 | 24 | 2 | 24 | 2 | 8 | 10×1 | 250 | PET | 12.5 | 5 | 324.5 | 100 | 99 | 35(no peel) | HF-2 |
| comparative example 2-5 | 24 | 10 | 24 | 10 | 42 | 10×1 | 250 | PET | 12.5 | 5 | 340.5 | 100 | 99 | 35(no peel) | HF-2 |
| comparative example 2-6 | 24 | 10 | 24 | 10 | 42 | 10×1 | 500 | PET | 25 | 5 | 603 | 100 | 100 | 15(no peel) | HF-2 |
| comperative example 2-7 | 24 | 10 | 24 | 10 | 42 | 10×1 | 250 | PET | 1.25 | 0.5 | 329.3 | 100 | 100 | 3(peel) | HF-1 |

As is apparent from Table 3, Examples 2-1 to 2-15 are evaluated according to UL 94 HF-1 in the flame retardancy test, whereas Comparative Example 2-1 shows insufficient flame retardancy of the activated carbon layer since the thermoplastic resin binder particles are of polyethylene and the binder particles become a flammable substance, Comparative Example 2-2 shows dropping off of the activated carbon at the time of combustion since the cover material was only on one side of the activated carbon layer, Comparative Examples 2-3 and 2-4 are insufficient in a flame-retardant effect to the cover material, Comparative Example 2-5 does not meet the above-mentioned ratio of the carbonized fibers, and Comparative Example 2-6 has the weight per unit area of activated carbon beyond the upper and lower limits, and thus, these comparative examples do not have flame retardancy satisfying UL Standard 94 HF-1. Comparative Example 2-7 has thermoplastic resin binder particles beyond the lower limit, is insufficient in adhesiveness, and causes peeling at the time of sheet formation.

## INDUSTRIAL APPLICABILITY

Since being excellent in removability of hazardous gas component and flame retardancy, the flame-retardant deodorizing filter of the present invention can be preferably used as a flame-retardant deodorizing filter for removing a hazardous gas component contained in a discharge gas of an electronic appliance such as a copy machine, a printer, a multi-functional OA appliance, a computer, a projector, or a POD printer, and as a flame-retardant deodorizing filter to be used for a refrigerator, a deodorization apparatus for toilets, or the like, and the like.

## DESCRIPTION OF THE NUMERALS

| | |
|---|---|
| 1 | Activated carbon particle |
| 2 | Thermoplastic resin powder |
| 3 | Melting fiber |
| A | Activated carbon layer |
| B | Fabric |

## Claims

1. A flame-retardant deodorizing filter comprising an activated carbon layer and a fabric formed on both sides thereof, wherein the fabric has a fiber ratio of one or more kinds selected from cellulose fibers, polyvinyl alcohol fibers, polyacrylonitrile fibers, and phenol fibers in 30% or more; a phosphorus-based flame retardant is contained in an amount of 10 to 70% by weight relative to the fabric weight; an adhesive layer is present between the activated carbon layer and the fabric; the activated carbon particles are firmly adhered by thermoplastic resin binder particles; and the weight per unit area of activated carbon is 50 to 400 g/m$^2$.

2. The flame-retardant deodorizing filter according to claim 1, wherein the activated carbon layer contains the thermoplastic resin binder particles in an amount of 1 to 10% by weight relative to the activated carbon weight mixed with the activated carbon.

3. A flame-retardant deodorizing filter comprising an activated carbon layer and a fabric formed on both sides thereof, wherein the fabric has a fiber ratio of one or more kinds selected from cellulose fibers, polyvinyl alcohol fibers, polyacrylonitrile fibers, and phenol fibers in 30% or more; a phosphorus-based flame retardant is contained in an amount of 10 to 80% by weight relative to the fabric weight; the activated carbon particles are firmly adhered by polyester resin or polyamide resin binder particles; and the weight per unit area of activated carbon is 50 to 400 g/m$^2$.

4. The flame-retardant deodorizing filter according to claim 3, wherein the activated carbon layer contains the polyester resin or polyamide resin binder particles in an amount of 1 to 15% by weight relative to the activated carbon weight mixed with the activated carbon.

FIG.1

FIG.2

FIG.3

<table>
<tr><td colspan="2" align="center">**INTERNATIONAL SEARCH REPORT**</td><td>International application No.<br>PCT/JP2011/068356</td></tr>
</table>

A. CLASSIFICATION OF SUBJECT MATTER
*B01D53/38*(2006.01)i, *A61L9/01*(2006.01)i, *A61L9/16*(2006.01)i, *B01D53/81*
(2006.01)i, *B01J20/20*(2006.01)i

According to International Patent Classification (IPC) or to both national classification and IPC

B. FIELDS SEARCHED

Minimum documentation searched (classification system followed by classification symbols)
B01D53/38, A61L9/01, A61L9/16, B01D53/81, B01J20/20

Documentation searched other than minimum documentation to the extent that such documents are included in the fields searched
| | | | |
|---|---|---|---|
| Jitsuyo Shinan Koho | 1922–1996 | Jitsuyo Shinan Toroku Koho | 1996–2011 |
| Kokai Jitsuyo Shinan Koho | 1971–2011 | Toroku Jitsuyo Shinan Koho | 1994–2011 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 2009-202150 A  (Japan Vilene Co., Ltd.),<br>10 September 2009 (10.09.2009),<br>paragraphs [0036], [0071] to [0078]; fig. 4<br>(Family: none) | 1-4 |
| Y | WO 2009/041257 A1  (Toray Industries, Inc.),<br>02 April 2009 (02.04.2009),<br>paragraph [0083]<br>& US 2010/0212506 A1    & EP 2206544 A1 | 1-4 |
| Y | JP 2009-61445 A  (Toray Industries, Inc.),<br>26 March 2009 (26.03.2009),<br>paragraphs [0047], [0048], [0073] to [0077]<br>(Family: none) | 1-4 |

☒ Further documents are listed in the continuation of Box C.   ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>02 November, 2011 (02.11.11) | Date of mailing of the international search report<br>15 November, 2011 (15.11.11) |
|---|---|
| Name and mailing address of the ISA/<br>Japanese Patent Office | Authorized officer |
| Facsimile No. | Telephone No. |

Form PCT/ISA/210 (second sheet) (July 2009)

25

**INTERNATIONAL SEARCH REPORT**

International application No.

PCT/JP2011/068356

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2004-50151 A  (Clariant (Japan) Kabushiki Kaisha), 19 February 2004 (19.02.2004), paragraph [0037] & WO 2004/009217 A1 | 2,4 |
| A | JP 2008-114109 A  (Japan Vilene Co., Ltd.), 22 May 2008 (22.05.2008), entire text; all drawings (Family: none) | 1-4 |

Form PCT/ISA/210 (continuation of second sheet) (July 2009)

**EP 2 604 327 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI1157467 A
- JP 8206550 A
- JP 7301434 A
- JP 8114109 A